(19) Europäisches Patentamt
European Patent Office
Office européen des brevets

(11) **EP 4 489 014 B1**

(12) **EUROPEAN PATENT SPECIFICATION**

(45) Date of publication and mention
of the grant of the patent:
**25.02.2026 Bulletin 2026/09**

(21) Application number: **23918049.0**

(22) Date of filing: **21.12.2023**

(51) International Patent Classification (IPC):
**G16C 60/00** (2019.01)     **C23C 8/20** (2006.01)
**C23C 8/22** (2006.01)

(52) Cooperative Patent Classification (CPC):
**C23C 8/20; C23C 8/22; G16C 60/00**

(86) International application number:
**PCT/CN2023/140459**

(87) International publication number:
**WO 2024/239634 (28.11.2024 Gazette 2024/48)**

(54) **METHOD FOR CALCULATING CARBON CONCENTRATION DISTRIBUTION DURING PULSE CARBURIZATION**

VERFAHREN ZUR BERECHNUNG DER KOHLENSTOFFKONZENTRATIONSVERTEILUNG WÄHREND DER IMPULSAUFKOHLUNG

PROCÉDÉ POUR CALCULER UNE DISTRIBUTION DE CONCENTRATION DE CARBONE PENDANT UNE CARBURATION PAR IMPULSIONS

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **24.05.2023   CN 202310591341**

(43) Date of publication of application:
**08.01.2025   Bulletin 2025/02**

(73) Proprietor: **Beijing Research Institute of Mechanical & Electrical Technology Co., Ltd. Cam Haidian District Beijing 100083 (CN)**

(72) Inventors:
• **CONG, Peiwu**
  **Beijing 100083 (CN)**
• **JIANG, Chao**
  **Beijing 100083 (CN)**
• **MA, Jingbo**
  **Beijing 100083 (CN)**
• **LU, Wenlin**
  **Beijing 100083 (CN)**
• **DU, Chunhui**
  **Beijing 100083 (CN)**
• **YAO, Jiawei**
  **Beijing 100083 (CN)**
• **CHEN, Xuyang**
  **Beijing 100083 (CN)**
• **YANG, Guangwen**
  **Beijing 100083 (CN)**

(74) Representative: **Lorenz & Kollegen
Lorenz & Kollegen Patentanwälte
Partnerschaftgesellschaft mbB
Alte Ulmer Straße 2
89522 Heidenheim (DE)**

(56) References cited:
CN-A- 102 063 110     CN-A- 103 177 193
CN-A- 110 457 834     CN-A- 116 564 452
JP-A- 2008 045 975

• GAO WEI MIN ET AL: "Integral Approach and Numerical Improvement to Calculate Carbon Concentration Profiles in Carburising", ADVANCED MATERIALS RESEARCH (DURNTEN-ZURICH, SWITZERLAND), vol. 264-265, 1 June 2011 (2011-06-01), pages 1494 - 1499, XP093273435, ISSN: 1662-8985, DOI: 10.4028/www.scientific.net/AMR.264-265.1494

## Description

### TECHNICAL FIELD

The present disclosure relates to the field of vacuum low-pressure carburizing, and in particular, to a method for calculating a carbon concentration distribution in a pulse carburizing process.

### BACKGROUND

Vacuum low-pressure carburizing originated from vacuum solid carburizing in the 1960s. In the 1970s, the vacuum solid carburizing had been gradually evolved into carburizing using a low-pressure gas and pulses, and dedicated vacuum carburizing equipment had emerged. In the 1990s, the concept of low-pressure carburizing had been clearly put forward, and it was experimentally demonstrated the advantages of acetylene as a carburizing medium in reducing carbon black production. After 2000, with the progress of research, a vacuum low-pressure carburizing process with acetylene as the carburizing medium and pulses as a working manner had been created. Along with worldwide problems such as environmental degradation and energy crisis, vacuum low-pressure carburizing, as a clean, efficient, and green thermal treatment technique, had received widespread attention.

Obtaining a carbon concentration distribution at a certain time in a carburizing process lays a foundation for describing and controlling a vacuum carburizing process. Unlike atmosphere carburizing, an oxygen probe cannot be used in the vacuum carburizing process and a carbon potential cannot be measured. It is thus impossible to obtain a real-time data feedback and realize real-time control. Therefore, the vacuum carburizing process relies more on the calculation of the carbon concentration distribution. Meanwhile, a boost-diffusion cyclic pulse process is employed for vacuum carburizing. Unlike atmosphere carburizing, a carbon concentration distribution cannot be calculated directly and rapidly to obtain information such as a surface carbon concentration and a carburized layer depth for guiding vacuum carburizing process control.

A method employed currently is a numerical calculation method. An equation (e.g., Fick law) may be discretized in space and time by using a finite element method or a finite difference method, and then is solved to obtain a carbon concentration distribution at a certain time in a carburizing process. However, the numerical calculation method may take a long time for calculation and require a lot of calculation resources, and cannot give a result in real time. Meanwhile, due to mathematical simplification of the numerical method, a systemic error may be produced, resulting in low calculation accuracy.

Gao et al. (GAO WEI MIN ET AL: "Integral Approach and Numerical Improvement to Calculate Carbon Concentration Profiles in Carburising", ADVANCED MATERIALS RESEARCH (DURNTEN-ZURICH, SWITZERLAND), vol. 264-265, 1 June 2011 (2011-06-01), pages 1494-1499, XP093273435, ISSN: 1662-8985, DOI: 10.4028/www.scientific.net/AMR. 264-265.1494) disclose an integral approach and numerical improvement to calculate carbon concentration profiles in carburising. The carbon diffusion in steel, where the carbon diffusivity varies with the carbon content, was solved with the integral methods under the third boundary condition. The variation of carbon diffusivity in steel with the carbon content was described with two different functions, linear dependence and exponential dependence. The integral approximation for both cases was improved with the numerical computation to more accurately predict the carbon profiles. The integral solution is more accurate than the formulation based on the assumption of a constant diffusivity or those based on the assumption of a constant diffusivity and/or constant carbon content at part surface. It is also more easily used in practice than the numerical method to describe the carburising process and predict the carbon content at steel surface and carbon profiles in treated layer.

### SUMMARY

For the defects in the prior art, an objective of the present disclosure is to provide a method for calculating a carbon concentration distribution in a pulse carburizing process. The method may obtain a carbon concentration distribution of each process during a pulse carburizing process and accurately describe a vacuum low-pressure carburizing process. The method is high in calculation accuracy and short in calculation time.

In order to achieve the above objective, the present disclosure adopts the following technical solutions:
A method for calculating a carbon concentration distribution in a pulse carburizing process includes:

step 1, when a carburizing process begins, obtaining a position where a carbon concentration first reaches a matrix carbon concentration $C_b$ from a surface to a core part, where a distance of the position from the surface is a carburized layer depth F;

step 2, selecting a positive number L and constructing an auxiliary function within an interval [-L, 0];

step 3, expanding a piecewise function composed of the auxiliary function and an initial carbon concentration distribution into a Fourier series within an interval [-L, F] such that a value $M(z)$ of a carbon concentration distribution within an interval [0, +∞] is in a form as shown by Formula (1):

$$M(\mathrm{z}) = \begin{cases} \sum_{n=-N}^{N} a_n \exp\left( i\frac{\pi n}{L} z \right), 0 \leq z \leq F \\ C_b, z > F \end{cases} \quad (1),$$

where z represents a carburized layer depth; $M(z)$ represents a carbon concentration after Fourier series expansion; $a_n$ represents a coefficient of the Fourier series; and $N$ represents a positive integer;

step 4, substituting $M(z)$ into an integral equation of Green's function method for integral calculation of a carbon concentration distribution of the current carburizing process, where the integral equation is as shown by Formula (2):

$$C(x,t) = \int_{-\infty}^{+\infty} G(x,z,t,0)M(z)dz + \int_{\tau=0}^{t} G(x,0,t,\tau)S(\tau)d\tau \quad (2),$$

where $G(x,z,t,0)$ and $G(x,0,t,\tau)$ both represent Green's functions, which are expressions when $\tau$ is 0 and z is 0 in the Green's function $G(x,z,t,\tau)$, respectively; $M(z)$ represents a carbon concentration distribution at the beginning of the carburizing process; $S(\tau)$ represents a boundary function; $C(x,t)$ represents the carbon concentration distribution of the current carburizing process; $x$ represents a carburized layer depth; $t$ represents a time consumed by the current carburizing process; $\tau$ represents an instantaneous time point of the current carburizing process, of which a value range is from 0 to $t$; and z represents a carburized layer depth; and

step 5, for each carburizing process, repeating the calculation processes of step 1 to step 4 to obtain carbon concentration distributions of all carburizing processes.

On the basis of the above solution,

the auxiliary function constructed in step 2 may be specifically a $Q$-term one-variable polynomial function in a form as shown by Formula (3):

$$K(z) = \sum_{q=0}^{Q-1} c_q z^q \quad (3)$$

where z represents a carburized layer depth within the interval [-L, 0]; and $c_q$ represents a coefficient corresponding to the term of $z$ to the power of $q$.

On the basis of the above solution,

the auxiliary function constructed in step 2 may be specifically a four-term one-variable polynomial function in a form as shown by Formula (4):

$$K(z) = \sum_{p=0}^{3} c_p z^p \quad (4);$$

where four equations are established according to four relationships: a value of the auxiliary function at a position -L is the matrix carbon concentration, a derivative value at the position -L is 0, a value at a position 0 is equal to a value of a carbon concentration distribution at the position 0, and a derivative value at the position 0 is equal to a derivative value of the carbon concentration distribution at the position 0; and four unknown coefficients $c_0$, $c_1$, $c_2$, and $c_3$ are ascertained, as shown in Formula (5):

$$c_0 = C(z = 0^+)$$

$$c_1 = \frac{dC(z)}{dz}\bigg|_{z=0^+}$$

$$c_2 = \frac{3C_b - 3c_0 + 2c_1 F}{F^2}$$

$$c_3 = \frac{2c_2 F - c_1}{3F^2} \tag{5},$$

where $C(z)$ represents a carbon concentration distribution when the current carburizing process begins; $z$ represents the carburized layer depth; and $C_b$ represents the matrix carbon concentration (wt.%).

On the basis of the above solution,

the auxiliary function constructed in step 2 may be specifically:
a five-term one-variable polynomial function in a form as shown by Formula (6):

$$K(z) = \sum_{p=0}^{4} c_p z^p \tag{6},$$

where five unknown coefficients are included; five equations are established according to five relationships: a value of the auxiliary function at a position -L is the matrix carbon concentration, a derivative value at the position -L is 0, a value at a position 0 is equal to a value of a carbon concentration distribution at the position 0, a derivative value at the position 0 is equal to a derivative value of the carbon concentration distribution at the position 0, and a function value at a position $H$mm is $C_f$, and the five unknown coefficients $c_0$, $c_1$, $c_2$, $c_3$, and $c_4$ are ascertained, as shown in Formula (7); $H$ is any negative number within an interval (-L, 0), and $C_f$ is any set value:

$$c_0 = C(z = 0^+)$$

$$c_1 = \frac{dC(z)}{dz}\bigg|_{z=0^+}$$

$$c_4 L^4 - c_3 L^3 + c_2 L^2 - c_1 L + c_0 = C_b$$

$$4c_4 L^3 + 3c_3 L^2 + 2c_2 L + c_1 = 0$$

$$c_4 H^4 - c_3 H^3 + c_2 H^2 - c_1 H + c_0 = C_f \tag{7},$$

where $C(z)$ represents a carbon concentration distribution when the current carburizing process begins; $z$ represents the carburized layer depth; and $C_b$ represents the matrix carbon concentration (wt.%).

On the basis of the above solution,

in step 4, if the current carburizing process is a diffusion process, the integral equation is as shown by Formula (8):

$$G(x,z,t,\tau) = \frac{1}{\sqrt{4D\pi(t-\tau)}}\left\{\exp\left[-\frac{(x-z)^2}{4D(t-\tau)}\right]+\exp\left[-\frac{(x+z)^2}{4D(t-\tau)}\right]\right\} \quad (8);$$

and

the boundary function $S(\tau)$ is equal to 0 in this case;

where $D$ represents a diffusion coefficient.

On the basis of the above solution,

in step 4, if the current carburizing process is a boost process, the integral equation is as shown by Formula (9):

$$G(x,z,t,\tau) = \frac{1}{\sqrt{4D\pi(t-\tau)}}\left\{\exp\left[-\frac{(x-z)^2}{4D(t-\tau)}\right]+\exp\left[-\frac{(x+z)^2}{4D(t-\tau)}\right]\right\}$$
$$-B\exp\left[B(x+z)+\left(B\sqrt{D(t-\tau)}\right)^2\right]\mathrm{erfc}\left\{\frac{x+z}{2\sqrt{D(t-\tau)}}+B\sqrt{D(t-\tau)}\right\} \quad (9);$$

and

the boundary function $S(z)$ is equal to $\beta C_g$ in this case;

where $D$ represents a diffusion coefficient; $\beta$ represents a surface transfer coefficient; B=β/D.

On the basis of the above solution,

in step 4, if the current carburizing process is a linear rising process of a carbon potential, the integral equation is as shown by Formula (10):

$$G(x,z,t,\tau) = \frac{1}{\sqrt{4D\pi(t-\tau)}}\left\{\exp\left[-\frac{(x-z)^2}{4D(t-\tau)}\right]+\exp\left[-\frac{(x+z)^2}{4D(t-\tau)}\right]\right\}$$
$$-B\exp\left[B(x+z)+\left(B\sqrt{D(t-\tau)}\right)^2\right]\mathrm{erfc}\left\{\frac{x+z}{2\sqrt{D(t-\tau)}}+B\sqrt{D(t-\tau)}\right\} \quad (10)$$

the boundary function $S(\tau)$ is equal to $\beta[(C_g-C_b)\tau/t_0+C_b]$ in this case;

where $D$ represents a diffusion coefficient; $\beta$ represents a surface transfer coefficient; B=β/D; $C_g$ represents the carbon potential; $C_b$ represents the matrix carbon concentration; and $t_0$ represents a time for the carbon potential to rise from the matrix carbon concentration $C_b$ to $C_g$.

The present disclosure further provides a computer-readable storage medium, storing a computer program which, when executed by a processor, is caused to implement the method for calculating a carbon concentration distribution in a pulse carburizing process as described in the above technical solution.

The method for calculating a carbon concentration distribution in a pulse carburizing process described in the present disclosure has the following beneficial effects.

(1) The method employs different boundary conditions to describe a boost process and a diffusion process, and can calculate a carbon concentration distribution at a certain time in each process (the boost process or the diffusion process) during a pulse carburizing process, accurately describe a vacuum low-pressure carburizing process, and present a varying process of a surface carbon concentration and a carburized depth in the vacuum low-pressure carburizing process over time.

(2) Based on the method, a relationship of a surface transfer coefficient and a diffusion coefficient of a material with a carbon concentration distribution of a workpiece after carburizing may be established, whereby the surface transfer

# EP 4 489 014 B1

coefficient and the diffusion coefficient of a material can be calculated according to the performance of the workpiece after vacuum low-pressure carburizing. The Fourier series expansion method is employed in the present disclosure. An equilibrium may be achieved between calculation accuracy and efficiency by adjusting a number of sum terms. The effects of high calculation accuracy and high calculation efficiency may be achieved with finite sum terms. The method of the present disclosure may be embedded in a computer with low calculation performance such as an industrial personal computer as an underlying algorithm of industrial software.

(3) The method can realize accurate control on the pulse carburizing process. A flow rate of a carburizing process gas may be given based on a surface carbon concentration at a certain time in the boost process in combination with information such as a carburizing gas type and a surface area of a workpiece. High accuracy control on the vacuum low-pressure carburizing process is realized such that further reduction of a carburizing gas pressure is achieved, thereby reducing carbon deposit production, increasing a carbon utilization ratio of the carburizing gas, and improving clean and green technical characteristics of vacuum low-pressure carburizing.

(4) By the method, process times for different carburizing manners to reach a certain carburizing target may be given, and then a carburizing manner with a short process time may be selected, giving full play to efficient technical characteristics of vacuum low-pressure carburizing.

## BRIEF DESCRIPTION OF THE DRAWINGS

The present disclosure has the following drawings:

FIG. 1 shows carbon concentration distributions at different times in a third carburizing process (a boost process) obtained by a calculation method in the present disclosure in Example 1;
FIG. 2 shows carbon concentration distributions at different times in a fourth carburizing process (a diffusion process) obtained by a calculation method in the present disclosure in Example 1;
FIG. 3 shows a relationship of a surface carbon concentration and a time in a carburizing process in Example 1;
FIG. 4 shows relationship of a carburized layer depth and a time in a carburizing process in Example 1;
FIG. 5 shows a mass flow rate of a gas corresponding to a mass of carbon entering a workpiece per second in a carburizing process in Example 1;
FIG. 6 shows carbon concentration distributions at different times in a third carburizing process (a boost process) obtained by a calculation method in the present disclosure in Example 2; and
FIG. 7 shows carbon concentration distributions at different times in a fourth carburizing process (a boost process) obtained by a calculation method in the present disclosure in Example 2.

## DETAILED DESCRIPTION OF THE EMBODIMENTS

The technical solutions in the embodiments of the present disclosure are described clearly and completely below with reference to the accompanying drawings in the embodiments of the present disclosure. Apparently, the described embodiments are merely some rather than all of the embodiments of the present disclosure. This does not impose limitations on the present disclosure. All other examples obtained by a person of ordinary skill in the art on the basis of the examples of the present disclosure without creative efforts shall fall within the protection scope of the present disclosure.

A method for calculating a carbon concentration distribution in a pulse carburizing process includes setting a carburized layer depth, constructing an auxiliary function, performing Fourier expansion, and performing integral calculation of a carbon concentration distribution.

The setting the carburized layer depth refers to setting a carburized layer depth F, and a value of a carbon concentration distribution at a position F is a matrix carbon concentration.

The constructing the auxiliary function refers to selecting a positive number L to construct an auxiliary function within an interval [-L, 0], where a value of the auxiliary function at a position -L is the matrix carbon concentration, a derivative value at the position -L is 0, a value at a position 0 is equal to a value of a carbon concentration distribution at the position 0, and a derivative value at the position 0 is equal to a derivative value of the carbon concentration distribution at the position 0.

The Fourier expansion refers to expanding a piecewise function composed of the auxiliary function and a carbon concentration distribution into a Fourier series within an interval [-L, F].

The integral calculation of the carbon concentration distribution refers to substituting the resulting piecewise function composed of the Fourier series and the matrix carbon concentration into an integral equation of Green's function method to ascertain a carbon concentration distribution of next carburizing stage.

In order to more clearly show the technical solutions provided by the present disclosure and the technical effects produced, the method provided by the present disclosure will be described in detail below with specific examples.

Example 1:

A surface transfer coefficient of a workpiece is $4.2 \times 10^{-8}$ m/s; a diffusion coefficient is $1.2 \times 10^{-11}$ m²/s; a surface area of the workpiece is 1 m²; and a density of the workpiece is $7.8 \times 10^3$ kg/m³.

A time and a type of each process during a carburizing process are as shown in Table 1.

A matrix carbon concentration is 0.2 wt.%, and a carburizing gas is acetylene.

Table 1 Time and Type of Each Process During Carburizing Process of Example 1

| Process No. | Carburizing Process Type | Time (s) |
|---|---|---|
| 1 | Boost process | 511 |
| 2 | Diffusion process | 133 |
| 3 | Boost process | 122 |
| 4 | Diffusion process | 173 |
| 5 | Boost process | 106 |
| 6 | Diffusion process | 209 |
| 7 | Boost process | 98 |
| 8 | Diffusion process | 243 |
| 9 | Boost process | 94 |
| 10 | Diffusion process | 276 |
| 11 | Boost process | 91 |
| 12 | Diffusion process | 309 |
| 13 | Boost process | 89 |
| 14 | Diffusion process | 342 |
| 15 | Boost process | 87 |
| 16 | Diffusion process | 1986 |

A method for calculating a carbon concentration distribution in a pulse carburizing process is carried out according to the following steps.

(1) An initial state is a non-carburized state of a workpiece, i.e., an internal carbon concentration of the workpiece is a matrix carbon concentration; and a carbon concentration distribution of a first boost process is calculated by using a method of calculating a carbon concentration distribution for atmosphere carburizing.

(2) A carburized layer depth is set: when a carburizing stage ends, a numerical algorithm is used to approach gradually from a surface to a core part to obtain a position where a carbon concentration reaches a core part carbon concentration for the first time from the surface to the core part, and a distance of the position from the surface is the carburized layer depth F.

(3) An auxiliary function is constructed by the following steps: select L to be equal to F; and construct a four-term one-variable polynomial function, including four unknown coefficients, where four equations are established according to four relationships: a value of the auxiliary function at a position -L is the matrix carbon concentration, a derivative value at the position -L is 0, a value at a position 0 is equal to a value of a carbon concentration distribution at the position 0, and a derivative value at the position 0 is equal to a derivative value of the carbon concentration distribution at the position 0; and the four unknown coefficients are ascertained. When the carburizing process is a diffusion process, the derivative value of the auxiliary function at the position 0 is 0.

$$c_0 = C(z = 0^+)$$

$$c_1 = \left. \frac{dC(z)}{dz} \right|_{z=0^+}$$

$$c_2 = \frac{3C_b - 3c_0 + 2c_1 F}{F^2}$$

$$c_3 = \frac{2c_2 F - c_1}{3F^2}$$

where $c_0$, $c_1$, $c_2$, and $c_3$ represent coefficients of the four-term one-variable polynomial function; $C(z)$ represents a carbon concentration distribution when the current carburizing process ends; $z$ represents the carburized layer depth; and $C_b$ represents the matrix carbon concentration (wt.%).

(4) Fourier expansion is performed: a piecewise function $M(z)$ composed of the auxiliary function and a carbon concentration distribution is expanded into a Fourier series within an interval [-F, F].

(5) Integral calculation of a carbon concentration distribution is performed: a number $N$ of sum terms of the Fourier series is selected to be 500, $M(z)$ being in the following form:

$$M(z) = \begin{cases} \displaystyle\sum_{n=-N}^{N} a_n \exp\left( i\frac{\pi n}{L} z \right), 0 \le z \le F \\ C_b, z > F \end{cases}$$

where $C_b$ represents the matrix carbon concentration (wt.%); z represents the carburized layer depth (in units of m); $M(z)$ represents a carbon concentration (wt.%) after curve fitting; and $a_n$ represents a coefficient of the piecewise function.

$M(z)$ is substituted into an integral equation of Green's function method.

The equation is as follows:

$$C(x,t) = \int_{-\infty}^{+\infty} G(x,z,t,0)M(z)dz + \int_{\tau=0}^{t} G(x,0,t,\tau)S(\tau)d\tau \quad \text{(Formula 1)}$$

where $G(x,z,t,0)$ and $G(x,0,t,\tau)$ represent Green's functions, which are expressions when $\tau$ is 0 and $z$ is 0 in Green's function $G(x,z,t,\tau)$, respectively;

$M(z)$ represents a carbon concentration distribution (wt.%) when a previous carburizing process ends;

$S(\tau)$ represents a boundary function;

$C(x,t)$ represents a carbon concentration distribution (wt.%) of the current carburizing process;

$x$ represents a carburized layer depth (m) of the current carburizing process;

$t$ represents a time (s) consumed by the current carburizing process;

$\tau$ represents an instantaneous time point of the current carburizing process, of which a value range is from 0 to $t$ and which is an integral intermediate variable; and

$z$ represents a carburized layer depth (m), which is an integral intermediate variable.

If the current carburizing process is the diffusion process, the equations are as follows:

$$G(x,z,t,\tau) = \frac{1}{\sqrt{4D\pi(t-\tau)}} \left\{ \exp\left[ -\frac{(x-z)^2}{4D(t-\tau)} \right] + \exp\left[ -\frac{(x+z)^2}{4D(t-\tau)} \right] \right\} \quad \text{(Formula 2)}$$

$$S(\tau) = 0 \quad \text{(Formula 3)}$$

If the current carburizing process is the boost process, the equation are as follows:

$$G(x,z,t,\tau) = \frac{1}{\sqrt{4D\pi(t-\tau)}}\left\{\exp\left[-\frac{(x-z)^2}{4D(t-\tau)}\right]+\exp\left[-\frac{(x+z)^2}{4D(t-\tau)}\right]\right\}$$

$$-B\exp\left[B(x+z)+\left(B\sqrt{D(t-\tau)}\right)^2\right]\mathrm{erfc}\left\{\frac{x+z}{2\sqrt{D(t-\tau)}}+B\sqrt{D(t-\tau)}\right\} \quad \text{(Formula 4)}$$

$$S(\tau) = \beta C_g \quad \text{(Formula 5)}$$

If the current carburizing process is a linear rising process of a carbon potential, the equations are as follows:

$$G(x,z,t,\tau) = \frac{1}{\sqrt{4D\pi(t-\tau)}}\left\{\exp\left[-\frac{(x-z)^2}{4D(t-\tau)}\right]+\exp\left[-\frac{(x+z)^2}{4D(t-\tau)}\right]\right\}$$

$$-B\exp\left[B(x+z)+\left(B\sqrt{D(t-\tau)}\right)^2\right]\mathrm{erfc}\left\{\frac{x+z}{2\sqrt{D(t-\tau)}}+B\sqrt{D(t-\tau)}\right\} \quad \text{(Formula 6)}$$

$$S(\tau) = \beta\left[\left(C_g - C_b\right)\frac{\tau}{t_0}+C_b\right] \quad \text{(Formula 7)}$$

where D represents a diffusion coefficient ($m^2$/s);
$\beta$ represents a surface transfer coefficient (m/s);
$B$:

$$B = \beta/D;$$

$C_g$ represents the carbon potential (wt.%);
$C_b$ represents the matrix carbon concentration (wt.%);
$t_0$ represents a time for the carbon potential to rise from the matrix carbon concentration $C_b$ to $C_g$.

An integral is solved so that the carbon concentration distribution $C(x,t)$ of the current carburizing process can be obtained.
(6) For each carburizing process, the calculation processes of step (2) to step (5) are repeated to obtain carbon concentration distributions of all carburizing processes.

Result analysis:

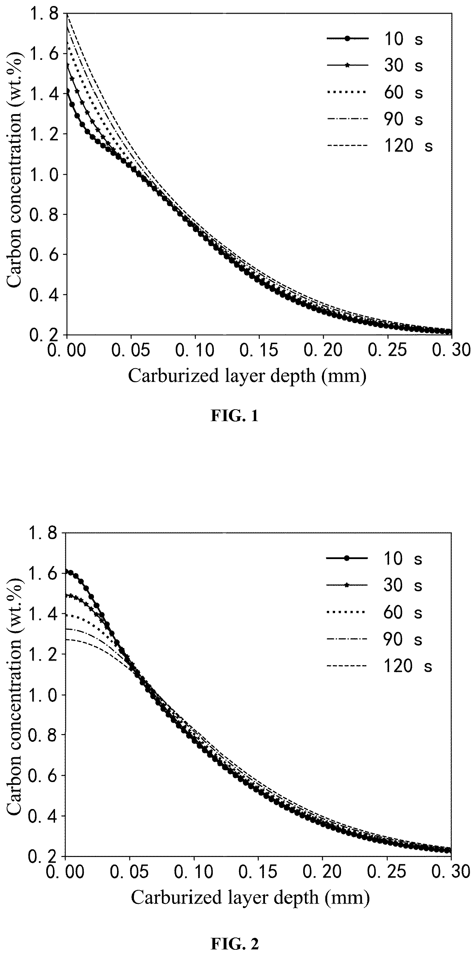

FIG. 1 shows carbon concentration distributions at different times in a third kind boundary condition (the boost process) obtained by the calculation method in the present disclosure. As can be seen from the figure, during the boost process, the carbon concentration within the carburized layer increases constantly with increasing carburizing time. The carbon concentration increases faster when getting closer to the surface. FIG. 2 shows carbon concentration distributions at different times in a second kind boundary condition (the diffusion process) obtained by the calculation method in the present disclosure. As can be seen from the figure, during the diffusion process, the carbon concentration at a position closer to the surface decreases constantly with increasing carburizing time, while the carbon concentration at a position closer to the core part increases constantly. FIG. 1 and FIG. 2 indicate that the calculation method in the present disclosure can effectively calculate the carbon concentration distribution during the carburizing process. Under the condition of single core calculation at a primary frequency 1.60 GHz, the whole calculation process takes 31 seconds, which is far less than 10-20 minutes for existing finite element software. A calculation error is characterized using a curvilinear integral, and a calculation error of a single step is less than 0.0001%, proving that the calculation method in the present disclosure has the characteristics of high calculation speed and high calculation accuracy.

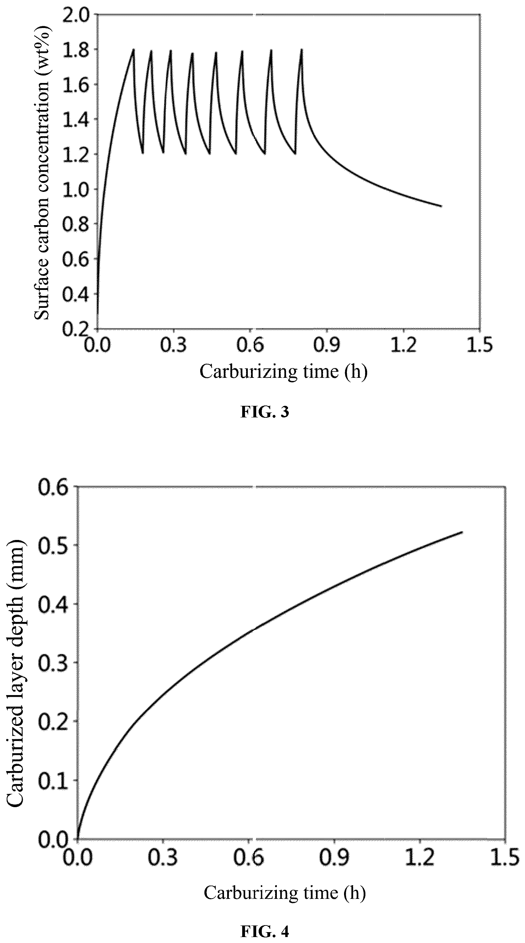

FIG. 3 shows a relationship of a surface carbon concentration and a time in a carburizing process. As can be seen from the figure, the surface carbon concentration increases in the boost process and decreases in the diffusion process. FIG. 4 shows relationship of a carburized layer depth and a time in a carburizing process. As can be seen from the figure, the carburized layer depth increases constantly with creasing time. FIG. 3 and FIG. 4 indicate that the calculation method in the present disclosure can give information such as a surface carbon concentration variation and a carburized layer depth variation in the carburizing process.

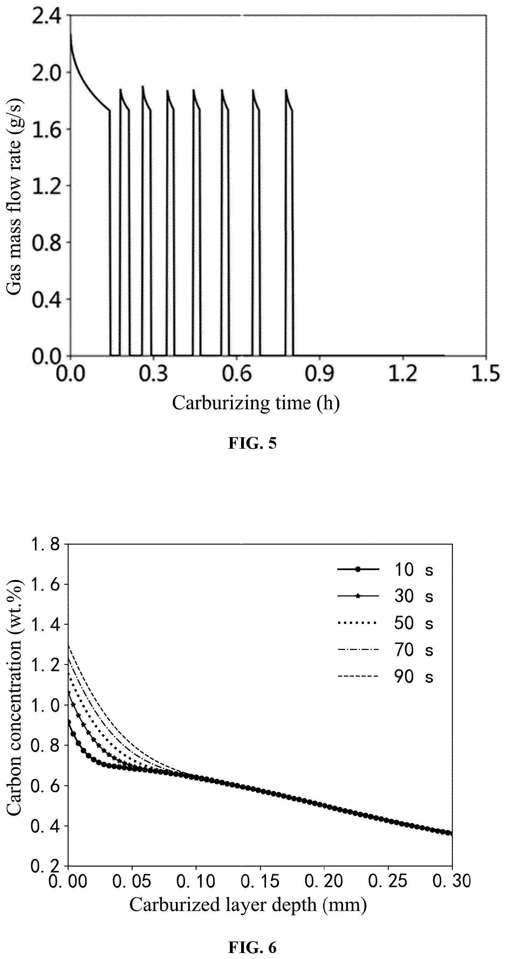

FIG. 5 shows a mass flow rate of a gas corresponding to a mass of carbon entering a workpiece per second in a carburizing process. When a technique of reducing a carburizing gas pressure and controlling a gas flow rate to reduce carbon deposit is employed, a gas mass flow rate that should be used in the carburizing process is in directly proportional to the mass of carbon entering the workpiece per second. FIG. 5 indicates that the calculation method in the present disclosure can give the gas mass flow rate required to realize carbon deposit reduction.

Example 2:

A surface transfer coefficient of a workpiece is $3.9 \times 10^{-8}$ m/s; a diffusion coefficient is $1.4 \times 10^{-11}$ m$^2$/s; a surface area of the workpiece is 1 m$^2$; and a density of the workpiece is $7.8 \times 10^3$ kg/m$^3$.

A time and a type of each process during a carburizing process are as shown in Table 2.

A matrix carbon concentration is 0.2 wt.%, and a carburizing gas is acetylene.

Table 2 Time and Type of Each Process During Carburizing Process of Example 2

| Process No. | Carburizing Process Type | Time (s) |
|---|---|---|
| 1 | Boost process | 589 |
| 2 | Diffusion process | 1119 |
| 3 | Boost process | 100 |
| 4 | Boost process | 197 |
| 5 | Diffusion process | 2073 |
| 6 | Boost process | 283 |
| 7 | Diffusion process | 3073 |

A method for calculating a carbon concentration distribution in a pulse carburizing process is carried out according to the following steps.

(1) An initial state is a non-carburized state of a workpiece, i.e., an internal carbon concentration of the workpiece is a matrix carbon concentration; and a carbon concentration distribution of a first boost process is calculated by using a method of calculating a carbon concentration distribution for atmosphere carburizing.

(2) A carburized layer depth F is set to 5 mm.

(3) An auxiliary function is constructed by the following steps: select L to be 6mm; and construct a five-term one-variable polynomial function, including five unknown coefficients, where five equations are established according to five relationships: a value of the auxiliary function at a position -L is the matrix carbon concentration, a derivative value at the position -L is 0, a value at a position 0 is equal to a value of a carbon concentration distribution at the position 0, a derivative value at the position 0 is equal to a derivative value of the carbon concentration distribution at the position 0, and a function value at a position -3mm (i.e., $H$=-3 mm) is 0.6wt.% (i.e., $C_f$=0.6wt.%) and the five unknown coefficients are ascertained.

Equations are as follows:

$$c_0 = C(z = 0^+)$$

$$c_1 = \left. \frac{dC(z)}{dz} \right|_{z=0^+}$$

$$c_4 L^4 - c_3 L^3 + c_2 L^2 - c_1 L + c_0 = C_b$$

$$4c_4 L^3 + 3c_3 L^2 + 2c_2 L + c_1 = 0$$

$$c_4 H^4 - c_3 H^3 + c_2 H^2 - c_1 H + c_0 = C_f$$

where $c_0$, $c_1$, $c_2$, $c_3$, and $c_4$ represent coefficients of the four-term one-variable polynomial function; $C(z)$ represents a carbon concentration distribution when the current carburizing process ends; $z$ represents the carburized layer depth; and $Cb$ represents the matrix carbon concentration (wt.%).

(4) Fourier expansion is performed: a piecewise function $M(z)$ composed of the auxiliary function and a carbon concentration distribution is expanded into a Fourier series within an interval [-L, F].

(5) Integral calculation of a carbon concentration distribution is performed: a number $N$ of sum terms of the Fourier series is selected to be 200, $M(z)$ being in the following form:

$$M(z) = \begin{cases} \sum_{n=-N}^{N} a_n \exp\left( i\frac{\pi n}{L} z \right), 0 \le z \le F \\ C_b, z > F \end{cases}$$

$M(z)$ is substituted into an integral equation of Green's function method.

The equation is as follows:

$$C(x,t) = \int_{-\infty}^{+\infty} G(x,z,t,0)M(z)dz + \int_{\tau=0}^{t} G(x,0,t,\tau)S(\tau)d\tau \qquad \text{(Formula 8)}$$

where $G(x,z,t,0)$ and $G(x,0,t,\tau)$ represent Green's functions, which are expressions when $\tau$ is 0 and $z$ is 0 in Green's function $G(x,z,t,\tau)$, respectively;
$M(z)$ represents a carbon concentration distribution (wt.%) when a previous carburizing process ends;
$S(\tau)$ represents a boundary function;
$C(x,t)$ represents a carbon concentration distribution (wt.%) of the current carburizing process;
$x$ represents a carburized layer depth (m) of the current carburizing process;
$t$ represents a time (s) consumed by the current carburizing process;
$\tau$ represent an instantaneous time point of the current carburizing process, of which a value range is from 0 to t and which is an integral intermediate variable; and
$z$ represents a carburized layer depth (m), which is integral intermediate variable.

If the current carburizing process is the diffusion process, the equations are as follows:

$$G(x,z,t,\tau) = \frac{1}{\sqrt{4D\pi(t-\tau)}} \left\{ \exp\left[ -\frac{(x-z)^2}{4D(t-\tau)} \right] + \exp\left[ -\frac{(x+z)^2}{4D(t-\tau)} \right] \right\} \qquad \text{(Formula 9)}$$

$$S(\tau) = 0 \qquad \text{(Formula 10)}$$

If the current carburizing process is the boost process, the equations are as follows:

$$G(x,z,t,\tau) = \frac{1}{\sqrt{4D\pi(t-\tau)}}\left\{\exp\left[-\frac{(x-z)^2}{4D(t-\tau)}\right]+\exp\left[-\frac{(x+z)^2}{4D(t-\tau)}\right]\right\}$$
$$-B\exp\left[B(x+z)+\left(B\sqrt{D(t-\tau)}\right)^2\right]\mathrm{erfc}\left\{\frac{x+z}{2\sqrt{D(t-\tau)}}+B\sqrt{D(t-\tau)}\right\} \quad \text{(Formula 11)}$$

$$S(\tau) = \beta C_g \quad \text{(Formula 12)}$$

If the current carburizing process is a linear rising process of a carbon potential, the equations are as follows:

$$G(x,z,t,\tau) = \frac{1}{\sqrt{4D\pi(t-\tau)}}\left\{\exp\left[-\frac{(x-z)^2}{4D(t-\tau)}\right]+\exp\left[-\frac{(x+z)^2}{4D(t-\tau)}\right]\right\}$$
$$-B\exp\left[B(x+z)+\left(B\sqrt{D(t-\tau)}\right)^2\right]\mathrm{erfc}\left\{\frac{x+z}{2\sqrt{D(t-\tau)}}+B\sqrt{D(t-\tau)}\right\} \quad \text{(Formula 13)}$$

$$S(\tau) = \beta\left[\left(C_g - C_b\right)\frac{\tau}{t_0} + C_b\right] \quad \text{(Formula 14)}$$

where D represents the diffusion coefficient (m$^2$/s);
$\beta$ represents the surface transfer coefficient (m/s);
$B$: $B=\beta/D$;
$C_g$ represents the carbon potential (wt.%);
$C_b$ represents the matrix carbon concentration (wt.%); and
$t_0$ represents a time for the carbon potential to rise from the matrix carbon concentration $C_b$ to $C_g$.

An integral is solved so that the carbon concentration distribution $C(x,t)$ of the current carburizing process can be obtained.

(6) For each carburizing process, the calculation processes of step (2) to step (5) are repeated to obtain carbon concentration distributions of all carburizing processes.

Result analysis:

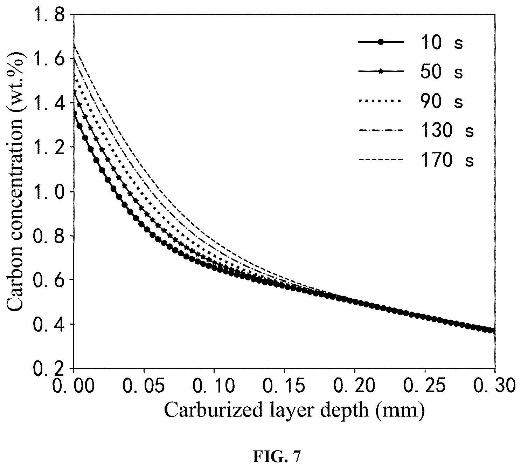

FIG. 6 shows carbon concentration distributions at different times in a third carburizing process (the boost process) obtained by the calculation method in the present disclosure. As can be seen from the figure, during the boost process, the carbon concentration within the carburized layer increases constantly with increasing carburizing time. FIG. 7 shows carbon concentration distributions at different times in a fourth carburizing process (the boost process) obtained by the calculation method in the present disclosure. FIG. 6 and FIG. 7 indicate that the calculation method of the present disclosure is not limited to boost-diffusion pulse cyclic process and is applicable to any combination of a finite number of boost and diffusion processes, and can be promoted widespread. Example 2 shows different values of F, L, and N from Example 1 and other construction method of the auxiliary function different from Example 1. Under the condition of single core calculation at a primary frequency 1.60 GHz, the whole calculation process takes about 14 seconds, which is far less than 10-20 minutes for existing finite element software. A calculation error is characterized using a curvilinear integral, and a calculation error of a single step is less than 0.001%. The number of sum terms used in this example is 200, which is reduced as compared to 500 in Example 1, and therefore, the calculation time is reduced, proving that the calculation method in the present disclosure has the characteristics of high calculation speed and high calculation accuracy.

The protection scope of the present disclosure should be subject to the protection scope defined by the claims. The information disclosed in the background section herein is only intended to deepen the understanding of the general background of the present disclosure, and should not be regarded as an acknowledgement or any form of suggestion that this information constitutes the prior art commonly known to those skilled in the art.

The content not described in detail in the description belongs to the prior art well known to those skilled in the art.

**Claims**

1. A computer-implemented method for calculating a carbon concentration distribution in a pulse carburizing process, **characterized in that** the method comprises:

   step 1, when a carburizing process begins, obtaining a position where a carbon concentration first reaches a matrix carbon concentration $C_b$ from a surface to a core part, wherein a distance of the position from the surface is a carburized layer depth F;
   step 2, selecting a positive number L and constructing an auxiliary function within an interval [-L, 0];
   step 3, expanding a piecewise function composed of the auxiliary function and an initial carbon concentration distribution into a Fourier series within an interval [-L, F] such that a value $M(z)$ of a carbon concentration distribution within an interval [0, +∞] is in a form as shown by Formula (1):

$$M(z) = \begin{cases} \sum_{n=-N}^{N} a_n \exp\left(i\frac{\pi n}{L}z\right), 0 \leq z \leq F \\ C_b, z > F \end{cases} \quad (1),$$

   wherein z represents a carburized layer depth; $M(z)$ represents a carbon concentration after Fourier series expansion; $a_n$ represents a coefficient of the Fourier series; and N represents a positive integer;
   step 4, substituting $M(z)$ into an integral equation of Green's function method for integral calculation of a carbon concentration distribution of the current carburizing process, wherein the integral equation is as shown by Formula (2):

$$C(x,t) = \int_{-\infty}^{+\infty} G(x,z,t,0)M(z)dz + \int_{\tau=0}^{t} G(x,0,t,\tau)S(\tau)d\tau \quad (2),$$

   wherein $G(x,z,t,0)$ and $G(x,0,t,\tau)$ both represent Green's functions, which are expressions when $\tau$ is 0 and $z$ is 0 in the Green's function $G(x,z,t,\tau)$, respectively; $M(z)$ represents a carbon concentration distribution at the beginning of the carburizing process; $S(\tau)$ represents a boundary function; $C(x,t)$ represents the carbon concentration distribution of the current carburizing process; $x$ represents a carburized layer depth; $t$ represents a time consumed by the current carburizing process; $\tau$ represents an instantaneous time point of the current carburizing process, which is an intermediate variable of integral calculation with a value range from 0 to $t$; and $z$ represents the carburized layer depth, which is an intermediate variable of integral calculation; and
   step 5, for each carburizing process, repeating the calculation processes of step 1 to step 4 to obtain carbon concentration distributions of all carburizing processes;
   wherein the auxiliary function constructed in step 2 is specifically a Q-term one-variable polynomial function in a form as shown by Formula (3):

$$K(z) = \sum_{q=0}^{Q-1} c_q z^q \quad (3),$$

   wherein $z$ represents a carburized layer depth within the interval [-L, 0]; and $c_q$ represents a coefficient corresponding to the term of $z$ to the power of $q$.

2. The method according to claim 1, wherein the auxiliary function constructed in step 2 is specifically a four-term one-variable

   polynomial function in a form as shown by Formula (4):

$$K(z) = \sum_{q=0}^{3} c_q z^q \quad (4);$$

wherein four equations are established according to four relationships: a value of the auxiliary function at a position -L is the matrix carbon concentration, a derivative value at the position -L is 0, a value at a position 0 is equal to a value of a carbon concentration distribution at the position 0, and a derivative value at the position 0 is equal to a derivative value of the carbon concentration distribution at the position 0; and four unknown coefficients $c_0$, $c_1$, $c_2$, and $c_3$ are ascertained, as shown in Formula (5):

$$c_0 = C(z = 0^+)$$

$$c_1 = \left. \frac{dC(z)}{dz} \right|_{z=0^+}$$

$$c_2 = \frac{3C_b - 3c_0 + 2c_1 F}{F^2}$$

$$c_3 = \frac{2c_2 F - c_1}{3F^2} \quad (5),$$

wherein $C(z)$ represents a carbon concentration distribution when the current carburizing process begins; $z$ represents the carburized layer depth; and $C_b$ represents the matrix carbon concentration (wt.%).

3. The method according to claim 1, wherein the auxiliary function constructed in step 2 is specifically:

a five-term one-variable polynomial function in a form as shown by Formula (6):

$$K(z) = \sum_{q=0}^{4} c_q z^q \quad (6)$$

wherein five unknown coefficients are comprised; five equations are established according to five relationships: a value of the auxiliary function at a position -L is the matrix carbon concentration, a derivative value at the position -L is 0, a value at a position 0 is equal to a value of a carbon concentration distribution at the position 0, a derivative value at the position 0 is equal to a derivative value of the carbon concentration distribution at the position 0, and a function value at a position $H$mm is $C_f$, and the five unknown coefficients $c_0$, $c_1$, $c_2$, $c_3$, and $c_4$ are ascertained, as shown in Formula (7); $H$ is any negative number within an interval (-L, 0), and $C_f$ is any set value:

$$c_0 = C(z = 0^+)$$

$$c_1 = \left. \frac{dC(z)}{dz} \right|_{z=0^+}$$

$$c_4 L^4 - c_3 L^3 + c_2 L^2 - c_1 L + c_0 = C_b$$

$$4c_4 L^3 + 3c_3 L^2 + 2c_2 L + c_1 = 0$$

$$c_4 H^4 - c_3 H^3 + c_2 H^2 - c_1 H + c_0 = C_f \quad (7),$$

wherein $C(z)$ represents a carbon concentration distribution when the current carburizing process begins; $z$ represents the carburized layer depth; and $C_b$ represents the matrix carbon concentration (wt.%).

4. The method according to claim 1, wherein

in step 4, if the current carburizing process is a diffusion process, the integral equation is as shown by Formula (8):

$$G(x,z,t,\tau) = \frac{1}{\sqrt{4D\pi(t-\tau)}}\left\{\exp\left[-\frac{(x-z)^2}{4D(t-\tau)}\right]+\exp\left[-\frac{(x+z)^2}{4D(t-\tau)}\right]\right\} \quad (8);$$

and
the boundary function $S(\tau)$ is equal to 0;
wherein $D$ represents a diffusion coefficient.

5.  The method according to claim 1, wherein

in step 4, if the current carburizing process is a boost process, the integral equation is as shown by Formula (9):

$$G(x,z,t,\tau) = \frac{1}{\sqrt{4D\pi(t-\tau)}}\left\{\exp\left[-\frac{(x-z)^2}{4D(t-\tau)}\right]+\exp\left[-\frac{(x+z)^2}{4D(t-\tau)}\right]\right\}$$
$$-B\exp\left[B(x+z)+\left(B\sqrt{D(t-\tau)}\right)^2\right]\mathrm{erfc}\left\{\frac{x+z}{2\sqrt{D(t-\tau)}}+B\sqrt{D(t-\tau)}\right\} \quad (9);$$

and
the boundary function $S(\tau)$ is equal to $\beta C_g$;
wherein $D$ represents a diffusion coefficient; $\beta$ represents a surface transfer coefficient; B=$\beta$/D; $C_g$ represents a carbon potential.

6.  The method according to claim 1, wherein

in step 4, if the current carburizing process is a linear rising process of a carbon potential, the integral equation is as shown by Formula (10):

$$G(x,z,t,\tau) = \frac{1}{\sqrt{4D\pi(t-\tau)}}\left\{\exp\left[-\frac{(x-z)^2}{4D(t-\tau)}\right]+\exp\left[-\frac{(x+z)^2}{4D(t-\tau)}\right]\right\}$$
$$-B\exp\left[B(x+z)+\left(B\sqrt{D(t-\tau)}\right)^2\right]\mathrm{erfc}\left\{\frac{x+z}{2\sqrt{D(t-\tau)}}+B\sqrt{D(t-\tau)}\right\} \quad (10);$$

and
the boundary function $S(\tau)$ is equal to $\beta[(C_g-C_b)\tau/t_0+C_b]$;
wherein $D$ represents a diffusion coefficient; $\beta$ represents a surface transfer coefficient; B=$\beta$/D; $C_g$ represents the carbon potential; $C_b$ represents the matrix carbon concentration; and $t_0$ represents a time for the carbon potential to rise from the matrix carbon concentration $C_b$ to $C_g$.

7.  A computer-readable storage medium, storing a computer program, **characterized in that** when executed by a processor, the computer program is caused to implement the method for calculating a carbon concentration distribution in a pulse carburizing process according to any one of claims 1 to 6.

**Patentansprüche**

1.  Computerimplementiertes Verfahren zur Berechnung einer Kohlenstoffkonzentrationsverteilung in einem Impulskohlenstoffisierungsprozess (Impulsaufkohlung),
    **dadurch gekennzeichnet, dass** das Verfahren umfasst:

Schritt 1: Wenn ein Aufkohlungsprozess beginnt, Ermitteln einer Position, an der eine Kohlenstoffkonzentration zuerst eine Matrixkohlenstoffkonzentration $C_b$ von einer Oberfläche zu einem Kernteil erreicht, wobei ein Abstand der Position von der Oberfläche eine Aufkohlungslagentiefe F ist;

Schritt 2: Auswählen einer positiven Zahl L und Konstruieren einer Hilfsfunktion innerhalb eines Intervalls [-L, 0];

Schritt 3: Erweitern einer stückweisen Funktion, die aus der Hilfsfunktion und einer anfänglichen Kohlenstoffkonzentrationsverteilung besteht, zu einer Fourier-Reihe innerhalb eines Intervalls [-L, F], sodass ein Wert $M(z)$ einer Kohlenstoffkonzentrationsverteilung innerhalb eines Intervalls [0, +∞] die in Formel (1) gezeigte Form hat:

$$M(\text{z}) = \begin{cases} \sum_{n=-N}^{N} a_n \exp\left(i\frac{\pi n}{L}z\right), 0 \le z \le F \\ C_b, z > F \end{cases}$$ (1),

wobei z eine Aufkohlungslagentiefe darstellt; $M(z)$ eine Kohlenstoffkonzentration nach der Fourier-Reihenent-wicklung darstellt; $a_n$ einen Koeffizienten der Fourier-Reihe darstellt; und N eine positive ganze Zahl darstellt;

Schritt 4, Ersetzen von $M(z)$ in einer Integralgleichung der Green-Funktionsmethode zur Integralberechnung einer Kohlenstoffkonzentrationsverteilung des aktuellen Aufkohlungsprozesses, wobei die Integralgleichung durch Formel (2) dargestellt wird:

$$C(x,t) = \int_{-\infty}^{+\infty} G(x,z,t,0)M(z)dz + \int_{\tau=0}^{t} G(x,0,t,\tau)S(\tau)d\tau$$ (2),

wobei $G(x,z,t,0)$ und $G(x,0,t,r)$ beide Green-Funktionen darstellen, die Ausdrücke sind, wenn $\tau$ 0 und z 0 in der Green-Funktion $G(x,z,t,\tau)$ sind; $M(z)$ eine Kohlenstoffkonzentrationsverteilung zu Beginn des Aufkohlungsproz-esses darstellt; $S(\tau)$ eine Randfunktion darstellt; $C(x,t)$ die Kohlenstoffkonzentrationsverteilung des aktuellen Aufkohlungsprozesses darstellt; $x$ eine Aufkohlungslagentiefe darstellt; t eine vom aktuellen Aufkohlungs-prozess verbrauchte Zeit darstellt; $\tau$ einen momentanen Zeitpunkt des aktuellen Aufkohlungsprozesses darstellt, der eine Zwischenvariable der Integralberechnung mit einem Wertebereich von 0 bis t ist; und z die Aufkohlungslagentiefe darstellt, die eine Zwischenvariable der Integralberechnung ist; und

Schritt 5: Für jeden Aufkohlungsprozess Wiederholen der Berechnungsprozesse von Schritt 1 bis Schritt 4, um die Kohlenstoffkonzentrationsverteilungen aller Aufkohlungsprozesse zu erhalten;

wobei die in Schritt 2 konstruierte Hilfsfunktion speziell eine Q-Term-Einvariablen-Polynomfunktion in der in Formel (3) gezeigten Form ist:

$$K(z) = \sum_{q=0}^{Q-1} c_q z^q$$ (3),

wobei z eine Aufkohlungslagentiefe innerhalb des Intervalls [-L, 0] darstellt; und $c_q$ einen Koeffizienten darstellt, der dem Term von z zur Potenz q entspricht.

2. Verfahren nach Anspruch 1, wobei die in Schritt 2 konstruierte Hilfsfunktion speziell eine viergliedrige einstellige Polynomfunktion in einer Form gemäß Formel (4) ist:

$$K(z) = \sum_{q=0}^{3} c_q z^q$$ (4);

wobei vier Gleichungen gemäß vier Beziehungen aufgestellt werden: Ein Wert der Hilfsfunktion an einer Position -L ist die Matrix-Kohlenstoffkonzentration, ein Ableitungswert an der Position -L ist 0, ein Wert an einer Position 0 ist gleich einem Wert einer Kohlenstoffkonzentrationsverteilung an der Position 0, und ein Ableitungswert an der Position 0 ist gleich einem Ableitungswert der Kohlenstoffkonzentrationsverteilung an der Position 0; und vier unbekannte Koeffizienten $c_0$, $c_1$, $c_2$ und $c_3$ werden wie in Formel (5) dargestellt ermittelt:

$$c_0 = C(z = 0^+)$$

$$c_1 = \left.\frac{dC(z)}{dz}\right|_{z=0^+}$$

$$c_2 = \frac{3C_b - 3c_0 + 2c_1 F}{F^2}$$

$$c_3 = \frac{2c_2 F - c_1}{3F^2} \quad (5),$$

wobei $C(z)$ eine Kohlenstoffkonzentrationsverteilung darstellt, wenn der aktuelle Aufkohlungsprozess beginnt; $z$ die Tiefe der aufgekohlten Schicht darstellt; und $C_b$ die Matrixkohlenstoffkonzentration (Gew.-%) darstellt.

**3.** Verfahren nach Anspruch 1, wobei die in Schritt 2 konstruierte Hilfsfunktion spezifisch ist:

eine fünfgliedrige einvariable Polynomfunktion in der in Formel (6) gezeigten Form:

$$K(z) = \sum_{q=0}^{4} c_q z^q \quad (6)$$

wobei fünf unbekannte Koeffizienten enthalten sind; fünf Gleichungen werden gemäß fünf Beziehungen aufgestellt: Ein Wert der Hilfsfunktion an einer Position -L ist die Matrixkohlenstoffkonzentration, ein Ableitungswert an der Position -L ist 0, ein Wert an einer Position 0 ist gleich einem Wert einer Kohlenstoffkonzentrationsverteilung an der Position 0, ein Ableitungswert an der Position 0 ist gleich einem Ableitungswert der Kohlenstoffkonzentrationsverteilung an der Position 0, und ein Funktionswert an einer Position Hmm ist $C_f$, und die fünf unbekannten Koeffizienten $c_0$, $c_1$, $c_2$, $c_3$ und $c_4$ werden wie in Formel (7) gezeigt ermittelt; H ist eine beliebige negative Zahl innerhalb eines Intervalls (-L, 0) und $C_f$ ist ein beliebiger festgelegter Wert:

$$c_0 = C(z = 0^+)$$

$$c_1 = \left.\frac{dC(z)}{dz}\right|_{z=0^+}$$

$$c_4 L^4 - c_3 L^3 + c_2 L^2 - c_1 L + c_0 = C_b$$

$$4c_4 L^3 + 3c_3 L^2 + 2c_2 L + c_1 = 0$$

$$c_4 H^4 - c_3 H^3 + c_2 H^2 - c_1 H + c_0 = C_f \quad (7),$$

wobei $C(z)$ eine Kohlenstoffkonzentrationsverteilung zu Beginn des aktuellen Aufkohlungsprozesses darstellt; $z$ die Tiefe der aufgekohlten Schicht darstellt; und $C_b$ die Matrixkohlenstoffkonzentration (Gew.-%) darstellt.

**4.** Verfahren nach Anspruch 1, wobei

in Schritt 4, wenn der aktuelle Aufkohlungsprozess ein Diffusionsprozess ist, die Integralgleichung wie in Formel (8) gezeigt lautet:

$$G(x,z,t,\tau) = \frac{1}{\sqrt{4D\pi(t-\tau)}}\left\{\exp\left[-\frac{(x-z)^2}{4D(t-\tau)}\right] + \exp\left[-\frac{(x+z)^2}{4D(t-\tau)}\right]\right\} \quad (8);$$

und
die Randfunktion S(r) gleich 0 ist;

wobei $D$ einen Diffusionskoeffizienten darstellt.

5. Verfahren nach Anspruch 1, wobei

in Schritt 4, wenn der aktuelle Aufkohlungsprozess ein Boost-Prozess ist, die Integralgleichung wie in Formel (9) gezeigt lautet:

$$G(x,z,t,\tau) = \frac{1}{\sqrt{4D\pi(t-\tau)}} \left\{ \exp\left[-\frac{(x-z)^2}{4D(t-\tau)}\right] + \exp\left[-\frac{(x+z)^2}{4D(t-\tau)}\right] \right\}$$

$$-B\exp\left[B(x+z)+\left(B\sqrt{D(t-\tau)}\right)^2\right]\mathrm{erfc}\left\{\frac{x+z}{2\sqrt{D(t-\tau)}}+B\sqrt{D(t-\tau)}\right\} \quad (9);$$

und
die Randfunktion $S(\tau)$ gleich $\beta C_g$ ist;
wobei D einen Diffusionskoeffizienten darstellt; $\beta$ einen Oberflächenübertragungskoeffizienten darstellt; $B=\beta/D$;
$C_g$ ein Kohlenstoffpotenzial darstellt.

6. Verfahren nach Anspruch 1, wobei

in Schritt 4, wenn der aktuelle Aufkohlungsprozess ein linear ansteigender Prozess eines Kohlenstoffpotentials ist, die Integralgleichung wie in Formel (10) gezeigt lautet:

$$G(x,z,t,\tau) = \frac{1}{\sqrt{4D\pi(t-\tau)}} \left\{ \exp\left[-\frac{(x-z)^2}{4D(t-\tau)}\right] + \exp\left[-\frac{(x+z)^2}{4D(t-\tau)}\right] \right\}$$

$$-B\exp\left[B(x+z)+\left(B\sqrt{D(t-\tau)}\right)^2\right]\mathrm{erfc}\left\{\frac{x+z}{2\sqrt{D(t-\tau)}}+B\sqrt{D(t-\tau)}\right\} \quad (10);$$

und
die Grenzfunktion $S(\tau)$ ist gleich $\beta[(C_g - C_b)\tau/t_0 + C_b]$;
wobei $D$ einen Diffusionskoeffizienten darstellt; 6 einen Oberflächenübertragungskoeffizienten darstellt; $B=\beta/D$;
$C_g$ das Kohlenstoffpotenzial darstellt; $C_b$ die Matrixkohlenstoffkonzentration darstellt; und $t_0$ eine Zeit darstellt, in der das Kohlenstoffpotenzial von der Matrixkohlenstoffkonzentration $C_b$ auf $C_g$ ansteigt.

7. Computerlesbares Speichermedium, das ein Computerprogramm speichert, **dadurch gekennzeichnet, dass** das Computerprogramm, wenn es von einem Prozessor ausgeführt wird, veranlasst wird, das Verfahren zur Berechnung einer Kohlenstoffkonzentrationsverteilung in einem Impulskohlenstoffisierungsprozess (Impulsaufkohlung) nach einem der Ansprüche 1 bis 6 zu implementieren.

**Revendications**

1. Procédé mis en œuvre par ordinateur pour calculer une distribution de concentration en carbone dans un processus de cémentation par impulsions, **caractérisé en ce que** le procédé comprend :

l'étape 1, lorsqu'un processus de cémentation commence, obtenir une position où la concentration en carbone atteint pour la première fois une concentration en carbone matricielle $C_b$ de la surface vers le cœur, la distance entre cette position et la surface correspondant à la profondeur de la couche cémentée F ;
étape 2, sélectionner un nombre positif L et construire une fonction auxiliaire dans un intervalle [-L, 0] ;
étape 3, développer une fonction par morceaux composée de la fonction auxiliaire et d'une distribution initiale de la concentration en carbone en une série de Fourier dans un intervalle [-L, F] de telle sorte qu'une valeur $M(z)$ d'une distribution de la concentration en carbone dans un intervalle [0, +∞] soit sous la forme indiquée par la

formule (1) :

$$M(z) = \begin{cases} \sum_{n=-N}^{N} a_n \exp\left( i\frac{\pi n}{L} z \right), 0 \le z \le F \\ C_b, z > F \end{cases}$$ (1),

où z représente une profondeur de couche cémentée ; $M(z)$ représente une concentration en carbone après développement en série de Fourier ; $a_n$ représente un coefficient de la série de Fourier ; et N représente un entier positif ;

étape 4, substitution de $M(z)$ dans une équation intégrale de la méthode de la fonction de Green pour le calcul intégral d'une distribution de concentration en carbone du processus de cémentation actuel, dans laquelle l'équation intégrale est représentée par la formule (2) :

$$C(x,t) = \int_{-\infty}^{+\infty} G(x,z,t,0) M(z) dz + \int_{\tau=0}^{t} G(x,0,t,\tau) S(\tau) d\tau$$ (2),

où $G(x,z,t,0)$ et $G(x,0,t,r)$ représentent toutes deux des fonctions de Green, qui sont des expressions lorsque $\tau$ est 0 et $z$ est 0 dans la fonction de Green $G(x,z,t,\tau)$, respectivement ; $M(z)$ représente une distribution de la concentration en carbone au début du processus de cémentation ; $S(\tau)$ représente une fonction limite ; $C(x,t)$ représente la distribution de la concentration en carbone du processus de cémentation en cours ; $x$ représente la profondeur de la couche cémentée ; $t$ représente le temps consommé par le processus de cémentation en cours ; $\tau$ représente un instant instantané du processus de cémentation en cours, qui est une variable intermédiaire du calcul intégral avec une plage de valeurs comprise entre 0 et $t$ ; et $z$ représente la profondeur de la couche cémentée, qui est une variable intermédiaire du calcul intégral ; et

étape 5, pour chaque processus de cémentation, répéter les processus de calcul des étapes 1 à 4 pour obtenir les distributions de concentration en carbone de tous les processus de cémentation ;

dans lequel la fonction auxiliaire construite à l'étape 2 est spécifiquement une fonction polynomiale à une variable de type Q sous la forme indiquée par la formule (3) :

$$K(z) = \sum_{q=0}^{Q-1} c_q z^q$$ (3),

où z représente une profondeur de couche cémentée dans l'intervalle [-L, 0] ; et $c_q$ représente un coefficient correspondant au terme de z à la puissance q.

2. Procédé selon la revendication 1, dans lequel la fonction auxiliaire construite à l'étape 2 est spécifiquement une fonction polynomiale à quatre termes et à une variable

sous la forme indiquée par la formule (4) :

$$K(z) = \sum_{q=0}^{3} c_q z^q$$ (4) ;

dans laquelle quatre équations sont établies selon quatre relations : une valeur de la fonction auxiliaire à une position -L est la concentration matricielle en carbone, une valeur dérivée à la position -L est 0, une valeur à une position 0 est égale à une valeur d'une distribution de concentration en carbone à la position 0, et une valeur dérivée à la position 0 est égale à une valeur dérivée de la distribution de concentration en carbone à la position 0 ; et quatre coefficients inconnus $c_0$, $c_1$, $c_2$ et $c_3$ sont déterminés, comme indiqué dans la formule (5) :

$$c_0 = C(z = 0^+)$$

$$c_1 = \frac{dC(z)}{dz}\bigg|_{z=0^+}$$

$$c_2 = \frac{3C_b - 3c_0 + 2c_1 F}{F^2}$$

$$c_3 = \frac{2c_2 F - c_1}{3F^2} \tag{5},$$

où $C(z)$ représente une distribution de concentration en carbone lorsque le processus de cémentation actuel commence ; $z$ représente la profondeur de la couche cémentée ; et $C_b$ représente la concentration en carbone de la matrice (en % en poids).

**3.** Procédé selon la revendication 1, dans lequel la fonction auxiliaire construite à l'étape 2 est spécifiquement :

une fonction polynomiale à cinq termes et une variable sous la forme indiquée par la formule (6) :

$$K(z) = \sum_{q=0}^{4} c_q z^q \tag{6}$$

dans laquelle cinq coefficients inconnus sont compris ; cinq équations sont établies selon cinq relations : une valeur de la fonction auxiliaire à une position -L est la concentration en carbone de la matrice, une valeur dérivée à la position -L est 0, une valeur à la position 0 est égale à une valeur d'une distribution de concentration en carbone à la position 0, une valeur dérivée à la position 0 est égale à une valeur dérivée de la distribution de concentration en carbone à la position 0, et une valeur de fonction à une position Hmm est $C_f$, et les cinq coefficients inconnus $c_0$, $c_1$, $c_2$, $c_3$ et $c_4$ sont déterminés, comme indiqué dans la formule (7) ; $H$ est un nombre négatif quelconque compris dans l'intervalle (-L, 0), et $C_f$ est une valeur quelconque :

$$c_0 = C(z = 0^+)$$

$$c_1 = \frac{dC(z)}{dz}\bigg|_{z=0^+}$$

$$c_4 L^4 - c_3 L^3 + c_2 L^2 - c_1 L + c_0 = C_b$$

$$4c_4 L^3 + 3c_3 L^2 + 2c_2 L + c_1 = 0$$

$$c_4 H^4 - c_3 H^3 + c_2 H^2 - c_1 H + c_0 = C_f \tag{7},$$

où $C(z)$ représente une distribution de concentration en carbone lorsque le processus de cémentation actuel commence ; $z$ représente la profondeur de la couche cémentée ; et $C_b$ représente la concentration en carbone de la matrice (en % en poids).

**4.** Procédé selon la revendication 1, dans lequel

à l'étape 4, si le processus de cémentation actuel est un processus de diffusion, l'équation intégrale est telle qu'indiqué par la formule (8) :

$$G(x,z,t,\tau) = \frac{1}{\sqrt{4D\pi(t-\tau)}}\left\{\exp\left[-\frac{(x-z)^2}{4D(t-\tau)}\right] + \exp\left[-\frac{(x+z)^2}{4D(t-\tau)}\right]\right\} \tag{8} ;$$

et
la fonction frontière S(r) est égale à 0 ;
où D représente un coefficient de diffusion.

**5.** Procédé selon la revendication 1, dans lequel

à l'étape 4, si le processus de cémentation actuel est un processus d'accélération, l'équation intégrale est telle que représentée par la formule (9) :

$$G(x,z,t,\tau) = \frac{1}{\sqrt{4D\pi(t-\tau)}} \left\{ \exp\left[ -\frac{(x-z)^2}{4D(t-\tau)} \right] + \exp\left[ -\frac{(x+z)^2}{4D(t-\tau)} \right] \right\}$$
$$- B\exp\left[ B(x+z) + \left( B\sqrt{D(t-\tau)} \right)^2 \right] \mathrm{erfc}\left\{ \frac{x+z}{2\sqrt{D(t-\tau)}} + B\sqrt{D(t-\tau)} \right\} \quad (9) ;$$

et
la fonction limite $S(\tau)$ est égale à $\beta C_g$ ;
où D représente un coefficient de diffusion ; $\beta$ représente un coefficient de transfert de surface ; $B=\beta/D$ ; $C_g$ représente un potentiel de carbone.

**6.** Procédé selon la revendication 1, dans lequel

à l'étape 4, si le processus de cémentation actuel est un processus d'augmentation linéaire du potentiel carbone, l'équation intégrale est telle que représentée par la formule (10) :

$$G(x,z,t,\tau) = \frac{1}{\sqrt{4D\pi(t-\tau)}} \left\{ \exp\left[ -\frac{(x-z)^2}{4D(t-\tau)} \right] + \exp\left[ -\frac{(x+z)^2}{4D(t-\tau)} \right] \right\}$$
$$- B\exp\left[ B(x+z) + \left( B\sqrt{D(t-\tau)} \right)^2 \right] \mathrm{erfc}\left\{ \frac{x+z}{2\sqrt{D(t-\tau)}} + B\sqrt{D(t-\tau)} \right\} \quad (10) ;$$

et
la fonction limite $S(\tau)$ est égale à $\beta[(C_g - C_b)\tau/t_0 + C_b]$ ;
où $D$ représente un coefficient de diffusion ; $\beta$ représente un coefficient de transfert de surface ; $B=\beta/D$ ; $C_g$ représente le potentiel carbone ; $C_b$ représente la concentration en carbone de la matrice ; et $t_0$ représente le temps nécessaire au potentiel carbone pour passer de la concentration en carbone de la matrice $C_b$ à $C_g$.

**7.** Support de stockage lisible par ordinateur, stockant un programme informatique, **caractérisé en ce que**, lorsqu'il est exécuté par un processeur, le programme informatique est amené à mettre en œuvre le procédé de calcul d'une distribution de concentration en carbone dans un processus de cémentation par impulsions selon l'une quelconque des revendications 1 à 6.

**FIG. 1**

**FIG. 2**

FIG. 3

FIG. 4

**FIG. 5**

**FIG. 6**

**FIG. 7**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GAO WEI MIN et al.** Integral Approach and Numerical Improvement to Calculate Carbon Concentration Profiles in Carburising. *ADVANCED MATERIALS RESEARCH*, 01 June 2011, vol. 264-265, 1494-1499